# EUROPÄISCHE PATENTSCHRIFT

(11) **EP 1 003 728 B1**
(45) Veröffentlichungstag und Bekanntmachung des Hinweises auf die Patenterteilung: **21.09.2011**
(21) Anmeldenummer: 98946343.5
(22) Anmeldetag: 14.08.1998
(51) Int. Cl.: C07D 239/56, A61K 31/505

(54) **2-(3-(4-(2-t-Butyl-6-trifluormethylpyrimidin-4-yl)piperazin-1-yl)propylmercapto)pyrimidin-4-ol-fumarat**
2-(3-(4-(2-t-Butyl-6-trifluoromethylpyrimidin-4-yl)piperazin-1-yl)propylmercapto)pyrimidin-4-ol-fumarate
Fumarate de 2-(3-(4-(2-t-butyl-6-trifluorométhylpyrimidin-4-yl)pipérazin-1-yl)propylmercapto)pyrimidin-4-ol

(30) Priorität: 14.08.1997 DE 19735410
(43) Veröffentlichungstag der Anmeldung: 31.05.2000
(60) Teilanmeldung: 10179278.6
(73) Patentinhaber: Abbott GmbH & Co. KG, 65205 Wiesbaden (DE)
(72) Erfinder: BLANK, Stefan, D-67056 Ludwigshafen (DE); STARCK, Dorothea, D-67059 Ludwigshafen (DE); TREIBER, Hans-Jörg, D-68782 Brühl (DE); KOSER, Stefan, D-67061 Ludwigshafen (DE); SCHÄFER, Bernd, D-76889 Dierbach (DE); THYES, Marco, D-67061 Ludwigshafen (DE); HÖGER, Thomas, D-68535 Edingen-Neckarhausen (DE)
(74) Vertreter: Reitstötter - Kinzebach
(86) Internationale Anmeldenummer: PCT/EP1998/005178
(87) Internationale Veröffentlichungsnummer: WO 1999/009015

(56) Entgegenhaltungen:
- WO-A-96/02519

## Beschreibung

2-{3-[4-(2-t-Butyl-6-trifluormethylpyrimidin-4-yl)piperazin-1-yl]propylmercapto}pyrimidin-4-ol-fumarat.

Die Erfindung betrifft das Fumarsäuresalz von 2-{3-[4-(2-t-Butyl-6-trifluormethylpyrimidin-4-yl)piperazin-1-yl]propylmer-capto}pyrimidin-4-ol und ein pharmazeutisches Mittel, das diese Verbindung enthält. Diese Verbindung besitzt wertvolle therapeutische Eigenschaften und ist insbesondere zur Behandlung von Erkrankungen brauchbar, die auf Dopamin-D₃-Liganden ansprechen.

Die WO 96/02519 beschreibt die erwähnte Verbindung in Form der freien Base der Formel 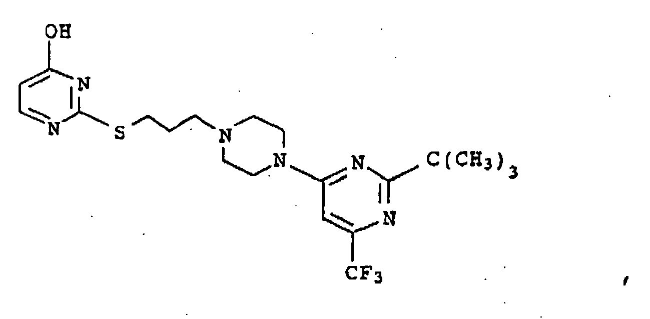 die ebenfalls zur Behandlung von Erkrankungen brauchbar ist, welche auf Dopamin-D₃-Liganden ansprechen. Ein Salz dieser Verbindung ist jedoch nicht offenbart.

Überraschenderweise wurde nun gefunden, daß das Säureadditionssalz dieser Verbindung mit Fumarsäure besondere Vorteile aufweist.

Gegenstand der vorliegenden Erfindung ist daher das Fumarsäure-salz von 2-{3-[4-(2-t-Butyl-6-trifluormethylpyrimidin-4-yl)piperazin-1-yl]propylmercapto}pyrimidin-4-ol sowie ein pharmazeutisches Mittel, welches diese Verbindung enthält.

Gegenstand der Erfindung sind auch die tautomeren Formen (Pyridonstruktur) des Fumarsäuresalzes.

Das Fumarsäuresalz besitzt sehr gute Affinität und hohe Selektivität gegenüber dem D₃-Rezeptor, d.h. es handelt sich um einen selektiven Dopamin-D₃-Rezeptor-Liganden, der regioselektiv im limbischen System angreift. Es besitzt eine selektivität Kᵢ D2/Kᵢ D₃ von 120 (vgl. WO 96/02519). Die Verbindung ist daher zur Behandlung von Erkrankungen brauchbar, die auf Dopamin-D₃-Liganden ansprechen, z.B. zur Behandlung von Erkrankungen des zentralen Nervensystems, insbesondere Schizophrenie, Depressionen, Neurosen und Psychosen. Außerdem ist sie zur Behandlung von Schlafstörungen, Übelkeit und als Antihistaminikum brauchbar. Untersuchungen zur Löslichkeit der Substanzen ergaben eine wesentlich höhere Löslichkeit des Salzes in Wasser im Vergleich zur freien Base. Dies ist für die Resorption bei oraler Anwendung und insbesondere bei parenteraler Anwendung von entscheidendem Vorteil.

Darüber hinaus ist das Fumarat in polaren Lösungsmitteln, wie C₁-C₆-Alkanolen, besser löslich als die freie Base. Aufgrund des veränderten Löslichkeitsverhaltens läßt es sich auch einfacher unter Vermeidung physiologisch nicht akzeptabler Lösungsmittel reinigen.

Die freie Base kann nach den in der WO 96/02519 beschriebenen allgemeinen Verfahren, vorzugsweise nach dem Verfahren (ii) hergestellt werden. Das Fumarat ist erhältlich durch Umsetzung der freien Base mit Fumarsäure in einem geeigneten Lösungsmittel, wie C₁-C₆-Alkanole, insbesondere Methanol, Ethanol, n-Propanol, Isopropanol und n-Butanol, einem Gemisch aus Wasser und einem der erwähnten Alkohole oder einem Ester, wie Essigsäureethylester. Im allgemeinen wird man bei erhöhter Temperatur arbeiten, damit das gewünschte Fumarat beim Abkühlen auskristalliert und auf einfache Weise isoliert werden kann. Die Fumarsäure wird im allgemeinen in äquimolaren Mengen oder mit einem geringen Überschuß von bis zu ca. 10 % zugegeben. Das Fumarat fällt auf diese Weise bereits in hoher Reinheit an. Es kann noch zusätzlich durch Ausrühren oder Umkristallisieren aus einem geeigneten Lösungsmittel, z. B. Wasser, einem der oben genannten Alkanole, Ester oder Gemischen davon, gereinigt werden.

Zur Behandlung der oben erwähnten Erkrankungen wird die erfindungsgemäße Verbindung in üblicher Weise oral oder parenteral (subkutan, intravenös, intramuskulär, intraperitoneal) verabreicht. Orale Verabreichung ist bevorzugt.

Die Dosierung hängt vom Alter, Zustand und Gewicht des Patienten sowie von der Applikationsart ab. In der Regel beträgt die tägliche Wirkstoffdosis etwa 10 bis 1000 mg pro Patient und Tag bei oraler Gabe und etwa 1 bis 500 mg pro Patient und Tag bei parenteraler Gabe.

Die Erfindung betrifft auch pharmazeutische Mittel, welche die erfindungsgemäße Verbindung enthalten. Diese Mittel liegen in den üblichen galenischen Applikationsformen in fester oder flüssiger Form vor, beispielsweise als Tabletten, Filmtabletten, Kapseln, Pulver, Granulate, Dragees, Suppositorien, Lösungen oder Sprays. Die Wirkstoffe können dabei mit den üblichen galenischen Hilfsmitteln, wie Tablettenbindemitteln, Füllstoffen, Konservierungsmitteln, Tablettensprengmitteln, Fließregulierungsmitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al., Pharmazeutische Technologie, Thieme-Verlag, Stuttgart, 1978). Die so erhaltenen Applikationsformen enthalten den Wirkstoff normalerweise in einer Menge von 1 bis 99 Gew.-%.

Die nachfolgenden Beispiele dienen zur Erläuterung der Erfindung.

### Beispiel 1

Herstellung des Fumarsäuresalzes von 2-{3-[4-[2-t-Butyl-6-trifluormethylpyrimidin-4-yl)-piperazin-1-yl]-propylmercapto]-pyrimidin-4-ol

2-t-Butyl-4-hydroxy-6-trifluormethyl-pyrimidin(1)

Die Ausgangsmaterialien sind literaturbekannt.

50 g (0,37 mol) 2,2-Dimethylpropanimidamid-hydrochlorid, gelöst in 200 ml Ethanol, wurden bei Raumtemperatur mit 66,6 g (0,37 mol) Natriummethylat (30%ig in Methanol) und nach weiteren 30 Minuten mit 52 g (0,28 mol) Trifluoracetessigsäureethylester versetzt. Nach 17 Stunden Rühren unter Rückfluß entfernte man das Lösungsmittel im Vakuum, versetzte den Rückstand mit 200 ml Wasser, acidifizierte bis pH 4 und isolierte den auskristallisierten Feststoff mittels Filtration.

| | |
|---|---|
| Ausbeute: 62,2 g | (98 % d.Th.) |
| C₉H₁₁F₃N₂O (MW 220) | Fp. 187-188°C |

2-t-Butyl-4-chlor-6-trifluormethylpyrimidin (2)

Zu einer Lösung von 60 g (0,27 mol) 2-t-Butyl-4-hydroxy-6-trifluormethylpyrimidin in 800 ml Dichlormethan wurden zunächst 86,5 ml Thionylchlorid, dann 8 ml DMF getropft und anschließend wurde zum Sieden unter Rückfluß erhitzt. Man entfernte die flüchtigen Bestandteile im Vakuum, nahm den Rückstand in 100 ml Dichlormethan auf, stellte mit gesättigter NaHCO₃-Lösung auf pH 7 ein und erhielt nach extraktiver Aufarbeitung 67 g (96 %) eines klaren Öles.

| | |
|---|---|
| Ausbeute 67 g | (96 % d. Th.) |
| C₉H₁₀ClF₃N₂ (MW 239) | |

2-t-Butyl-4-[piperazin-1-yl]-6-trifluormethyl-pyrimidin (3)

Zu einer Lösung von 129 g (1,5 mol) Piperazin in 500 ml Ethanol wurde eine Lösung von 60 g (0,25 mol) des oben beschriebenen Chlorpyrimidins, in 200 ml Ethanol bei Siedetemperatur innerhalb 2 h zugetropft und anschließend wurde weitere 6 h bei Siedetemperatur gerührt. Nach beendeter Reaktion wurde das Lösungsmittel im Vakuum entfernt und der Rückstand mit 2 1 Wasser versetzt. Das Produkt kristallisierte beim Abkühlen aus und wurde anschließend abgesaugt.

| | |
|---|---|
| Ausbeute: 56 g | (77 % d.Th.) |
| C₁₃H₁₉ClF₃N₄ (MW 288) | Fp. 78-80°C |

1H-NMR (250 MHz, CDCl₃): δ = 1,3 (s, 9H); 1,8 (s, 1H); 3,0 (m, 4H); 4,7 (m, 4H); 6,6 (s, 1H) ppm.

2-t-Butyl-4-[4-(3-chlorpropyl)-piperazin-1-yl]-6-trifluormethylpyrimidin (4)

Zu einer Mischung von 35,1 g (0,22 mol) 1-Brom-3-chlor-propan und 16,0 g (0,16 mol) Triethylamin in 90 ml THF wurden in der Siedehitze 43,2 g (0,15 mol) des vorstehend beschriebenen Piperazins, gelöst in 130 ml THF, getropft und 8 Stunden bei dieser Temperatur gerührt. Nach Abkühlen auf 4°C wurde von den anorganischen Salzen abfiltriert, die THF-Phase im Vakuum eingedampft und der Rückstand aus Isopropanol umkristallisiert.

| | |
|---|---|
| Ausbeute: 32,1 g | (61 % d.Th.) |
| C₁₆H₂₄ClF₃N₄ (MW 365) | Fp. 83-84°C |

1H-NMR (250 MHz, CDCl₃): δ = 1,3 (s, 9H); 1,9 (q, 2H); 2,5 (m, 6H); 3,7 (t, 2H); 3,8 (m, 4H); 6,6 (s, 1H) ppm.

2-{3-[4-[2-t-Butyl-6-trifluormethylpyrimidin-4-yl)-piperazin-1-yl]-propylmercapto}-pyrimidin-4-ol (5)

8,4 g (0,066 mol) Thiouracil, 1,6 g (0,066 mol) Lithiumhydroxid und 1,0 g (0,066 mol) Natriumjodid wurden in 200 ml DMF gelöst und auf 100°C erwärmt. Bei dieser Temperatur wurden 20,1 g (0,055 mol) der vorstehend beschriebenen Chlorbase, gelöst in 50 ml DMF, zugegeben und 30 min bei 100°C nachgerührt. Anschließend wurde mit 300 ml Natriumchloridlösung versetzt und 2 mal mit 200 ml Essigsäureethylester extrahiert. Die organische Phase wurde mit Natriumsulfat getrocknet und nach Filtration unter Vakuum eingedampft.

Der Rückstand wurde säulenchromatographisch (Kieselgel, Dichlormethan mit 1 - 4 % Methanol) gereinigt.

| | |
|---|---|
| Ausbeute: 18 g | (72 % d. Th). |
| C₂₀H₂₇F₃N₆OS (MW 457) | Fp. 138-140°C |

1H-NMR (270 MHz, DMSO-d₆) :δ = 1,3 (s, 9H); 1,8 (q, 2H); 2,4 (m, 6H); 3,3 (t, 2H); 3,75 (m, 4 H); 6,1 (d, 2H); 7,1 (s, 1H); 7,9 (2, 2H) ppm.

Herstellung des Fumarates (6):

4,56 g (0,01 mol) der vorstehend beschriebenen Base wurden in 25 ml Isopropanol heiß gelöst und eine heiße Lösung von 1,16 g (0,01 mol) Fumarsäure in 15 ml Isopropanol wurde zugegeben. Die beim Abkühlen auskristallisierte Substanz wurde abfiltriert, wobei 4,4 g der Titelverbindung als farblose Kristalle erhalten wurden.

| | |
|---|---|
| Ausbeute: 4,4 g | (76 % d. Th.) |
| C₂₀H₂₇F₃N₆OS x C₄H₄O₄ | (MW 573) Fp. 200 - 202°C |

1H-NMR (250 MHz, DMSO-d₆) : δ = 1,3 (s, 9H); 1,9 (q, 2H); 2,5 (m, 6H) ; 3,2 (t, 2H); 3,8 (mbr, 6H); 6,2 (d, 2H); 6,7 (s, 2H); 7,1 (s, 1H); 7,9 (d, 2H).

## Patentansprüche

1. Fumarsäuresalz von 2-{3-[4-(2-t-Butyl-6-trifluormethylpyrimidin-4-yl)piperazin-1-yl]propylmercapto}pyrimidin-4-ol der Formel 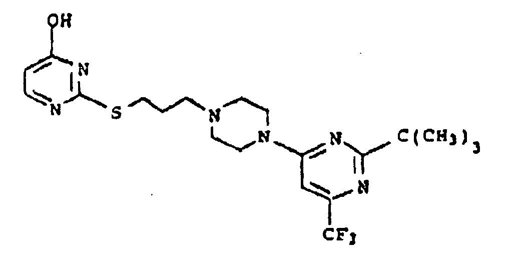 und die tautomeren Formen davon.

2. Pharmazeutisches Mittel, enthaltend das Fumarsäuresalz oder eine tautomere Form davon nach Anspruch 1, gegebenenfalls zusammen mit physiologisch akzeptablen Trägern und/oder Hilfsstoffen.

3. Verwendung des Fumarsäuresalzes oder einer tautomeren Form davon nach Anspruch 1 zur Herstellung eines pharmazeutischen Mittels zur Behandlung von Erkrankungen, die ausgewählt sind unter Schizophrenie, Depressionen, Neurosen, Psychosen Schlafstörungen und übelkeit, und als Antihistaminikum.

## Claims

1. Fumaric acid salt of 2-(3-(4-(2-t-butyl-6-trifluoromethylpyrimidin-4-yl)piperazin-1-yl)propylmercapto)pyrimidin-4-ol of formula 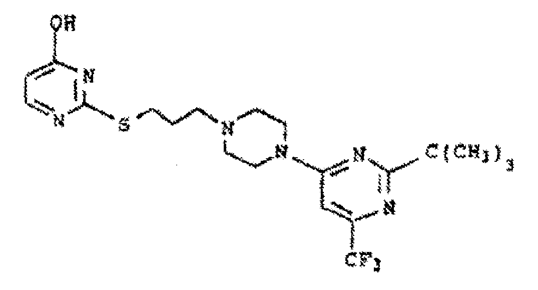 and the tautomeric forms thereof.

2. Pharmaceutical composition containing the fumaric acid salt or a tautomeric form thereof according to Claim 1, optionally together with physiologically acceptable carriers and/or excipients.

3. Use of the fumaric acid salt or of a tautomeric form thereof according to Claim 1 in the manufacture of a pharmaceutical composition for treating disorders selected from schizophrenia, depressions, neuroses, psychoses, sleep disturbances and nausea, and as antihistamine.

## Revendications

1. Sel d'acide fumarique avec le 2-{3-(4-(2-tert-butyl-6-trifluorométhylpyrimidin-4-yl)pipérazin-1-yl)-propylmercapto}pyrimidin-4-ol de formule 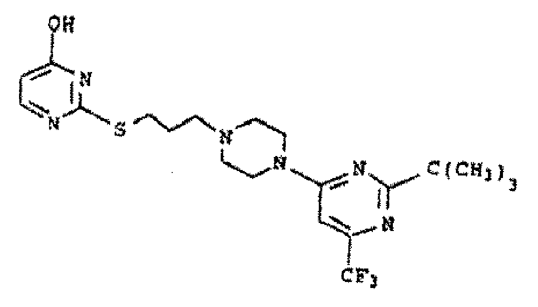 et les formes tautomères de celui-ci.

2. Composition pharmaceutique, contenant le sel d'acide fumarique ou une forme tautomère de celui-ci selon la revendication 1, éventuellement conjointement avec des véhicules et/ou adjuvants physiologiquement acceptables.

3. Utilisation du sel d'acide fumarique ou d'une forme tautomère de celui-ci selon la revendication 1, pour la préparation d'une composition pharmaceutique destinée au traitement de maladies qui sont choisies parmi la schizophrénie, les dépressions, les névroses, les psychoses, les troubles du sommeil et les nausées, et en tant qu'antihistaminique.
